# EUROPÄISCHE PATENTANMELDUNG

(11) **EP 1 013 646 A1**
(43) Veröffentlichungstag der Anmeldung: **28.06.2000**
(21) Anmeldenummer: 99123984.9
(22) Anmeldetag: 07.12.1999
(51) Int. Cl.: C07D 207/34

(54) **Verfahren zum Herstellen von 2,4-Dimethyl-3,5-bis-alkoxy-carbonyl-pyrrol**

(30) Priorität: 17.12.1998 DE 19858352
(71) Anmelder: BASF AKTIENGESELLSCHAFT, 67056 Ludwigshafen (DE)
(72) Erfinder: Gröning, Carsten, Dr., 68259 Mannheim (DE); Kemper, Reinhard, Dr., 69123 Heidelberg (DE); Frede, Markus, Dr., 69214 Eppelheim (DE); Ebel, Klaus, Dr., 68623 Lampertheim (DE)
(74) Vertreter: Isenbruck, Günter, Dr.

(57) **Zusammenfassung**

Es wird ein Verfahren zum Herstellen von 2,4-Dimethyl-3,5-bis-alkoxycarbonyl-pyrrol beschrieben, bei dem ein Acetessigsäurealkylester zum 2-Nitroso-acetessigsäurealkylester nitrosiert, die Nitrosoverbindung mit Wasserstoff in Gegenwart eines Edelmetallkatalysators zum Amin reduziert und die entstandene Aminoverbindung ohne Isolierung in Gegenwart des entsprechenden nicht nitrosierten Acetessigsäurealkylesters zum 2,4-Dimethyl-3,5-bis-alkoxycarbonyl-pyrrol kondensiert wird. Das Verfahren erlaubt es, unter milderen Bedingungen als im Stand der Technik zu arbeiten und die Bildung von Nebenprodukten zu vermindern.

## Beschreibung

Die Erfindung betrifft ein Verfahren zum Herstellen von 2,4-Dimethyl-3,5-bis-alkoxycarbonyl-pyrrol, bei dem ein Acetessigsäurealkylester in 2-Stellung nitrosiert, zur Aminoverbindung reduziert und dann zum 2,4-Dimethyl-3,5-bis-alkoxycarbonyl-pyrrol kondensiert wird.

Diese Verbindung ist neben anderen Einsatzmöglichkeiten ein wertvolles Zwischenprodukt bei der Synthese von 2,4-Dimethyl-pyrrol, das aus dem Dicarbonsäureester in bekannter Weise durch Verseifung und Decarboxylierung erhalten wird.

Die Synthese des 2,4-Dimethyl-3,5-bis-ethoxycarbonyl-pyrrols ist in Org. Syntheses, Coll. Vol. II, 202 - 204 (1943) beschrieben. Als Reduktionsmittel für die Nitrosogruppe wird elementares Zink eingesetzt. Die Ausbeute liegt bei maximal 64 %. Von Treibs et al. wird die Reduktion der Nitrosoverbindung mit Natriumdisulfat (III) (Natriumdithionit) in Chem. Ber. 90, 79 (1957) in 85 %iger Ausbeute beschrieben.

Beide Reduktionsmittel haben technologische Nachteile . Zink als Feststoff ist technisch nur unter höherem Aufwand zu handhaben. Die umweltgerechte Entsorgung oder auch Wiederaufarbeitung der anfallenden, Zinksalze enthaltenden Lösungen verursacht erhebliche Kosten. Auch der Einsatz von Natriumdithionit ergibt eine erhöhte Salzfracht und ist aus Umweltaspekten unerwünscht.

Aus den EP-A 170 214 und 078 545 ist die Hydrierung von nitrosierten 1,3-Di-ketonen bzw. von nitrosierten β-Keto-carbonsäureestern mit Wasserstoff in Gegenwart eines Palladiumkatalysators zu den entsprechenden Aminen bekannt. Wenn ein 2-Nitroso-acetessigester isoliert und dann in dieser Weise hydriert wird, entsteht im wesentlichen durch Kondensation von 2 Molekülen Aminoverbindung zum Sechsring bereits das 2,5-Dimethyl-3,6-bis-alkoxycarbonyl-dihydropyrazin oder dessen Hydrierungsprodukte. Eine Kondensation der Aminoverbindung mit unsubstitutiertem Acetessigester zum Pyrrolderivat ist somit nicht mehr möglich.

Aufgabe der Erfindung war es, ein Verfahren zum Herstellen von 2,4-Dimethyl-3,5-bis-alkoxycarbonyl-pyrrol vorzuschlagen, das das Produkt in hoher Ausbeute liefert und bei dem die anfallenden Nebenprodukte problemlos entsorgt werden können.

Die Erfindung geht aus von einem Verfahren zum Herstellen von 2,4-Dimethyl-3,5-bis-alkoxycarbonyl-pyrrol, bei dem ein Acetessigsäurealkylester zum 2-Nitroso-acetessigsäurealkylester nitrosiert, die Nitrosoverbindung zum Amin reduziert und das Amin zusammen mit unsubstituiertem Acetessigsäurealkylester unter Ringschluß zum 2,4-Dimethyl-3,5-bis-alkoxycarbonyl-pyrrol kondensiert wird.

Das erfindungsgemäße Verfahren ist dadurch gekennzeichnet, daß man die Nitrosoverbindung mit Wasserstoff in Gegenwart eines Edelmetallkatalysators hydriert und die entstandene Aminoverbindung ohne Isolierung in Gegenwart des entsprechenden nicht nitrosierten Acetessigsäurealkylesters zum 2,4-Dimethyl-3,5-bis-alkoxycarbonyl-pyrrol kondensiert.

Die Synthese verläuft nach dem folgenden Formelschema

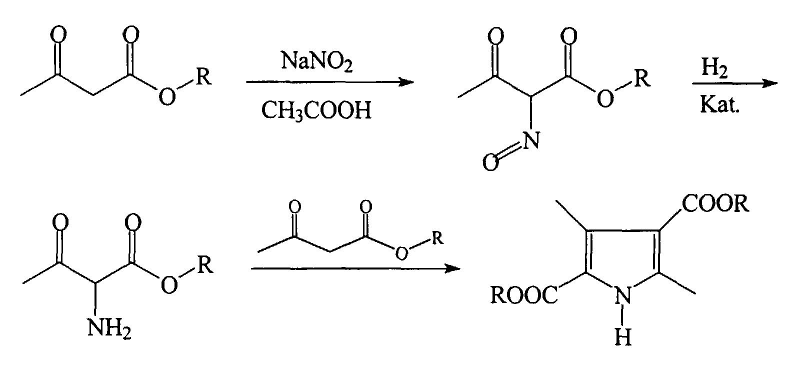

R bedeutet eine Alkylgruppe mit 1 bis 4 C-Atomen und ist bevorzugt Methyl oder Ethyl.

Als Edelmetallkatalysator kann ein Metall der Platingruppe, zum Beispiel Palladium oder Platin, auf einem Träger mit großer Oberfläche, zum Beispiel Aktivkohle, eingesetzt werden.

Es wurde nun gefunden, daß überraschenderweise ohne Isolierung der Nitrosoverbindung durch Hydrieren der nitrosierten Rohlösung in Anwesenheit von überschüssigem Acetessigsäurealkylester unmittelbar das gewünschte Pyrrolderivat erhalten wird.

Für die Synthese ist es daher wesentlich, daß die Nitrosierung mit einem Unterschuß, d.h. etwa 10 bis 50, bevorzugt 20 bis 40 % der stöchiometrischen Menge an Natriumnitrit erfolgt. Die Nitrosierung mit Natriumnitrit und Essigsäure in wäßriger Lösung erfolgt in üblicher Weise unter Kühlung auf eine Temperatur zwischen 0°C und Raumtemperatur, bevorzugt unterhalb 15°C.

Die katalytische Hydrierung wird normalerweise bei leicht erhöhter Temperatur, etwa im Bereich oberhalb Raumtemperatur bis 60°C, und unter einem Wasserstoffdruck von 5 bis 20 bar durchgeführt. Das entstandene 2,4-Dimethyl-3,5-bis-alkoxycarbonyl-pyrrol fällt aus der Lösung aus und kann abfiltriert werden.

Die folgenden Beispiele erläutern die Vorteile des erfindungsgemäßen Verfahrens. Mengenverhältnisse und Prozentangaben sind, wenn nichts anderes angegeben ist, in Gewichtseinheiten zu verstehen.

### Beispiel 1 (Vergleichsbeispiel)

Eine Lösung aus 70 g Acetessigsäuremethylester (0,60 mol) in 100 g Essigsäure wurde bei 5 bis 10°C mit einer Lösung von 50 g Natriumnitrit (0,72 mol) in 75 g Wasser nitrosiert. Die überschüssige salpetrige Säure wurde mit Harnstoff zerstört; die Vollständigkeit der Reaktion wurde mit Kaliumjodid-Stärkepapier geprüft. Die Lösung wurde mit Ether extrahiert, der Extrakt mit Natriumcarbonatlösung und Wasser gewaschen und der Ether im Vakuum abdestilliert. Es verblieben 30,8 g eines gelben Öls.

10 g des erhaltenen Öls wurden mit 2 g Pd/Aktivkohle (5 % Pd) in 100 ml Essigsäure bei 50°C unter 9 bar Wasserstoffdruck hydriert. Das feste Sediment wurde abfiltriert und das Filtrat mit 20g Acetessigsäuremethylester zum Sieden erhitzt. Das Filtrat wurde mit Natriumcarbonatlösung neutralisiert. Dabei fiel nicht das gewünschte 2,4-Dimethyl-3,5-bis-methoxycarbonyl-pyrrol aus. Die Lösung wurde mit Ether extrahiert und gaschromatographisch untersucht. Dabei wurden nur das 2,5-Dimethyl-3,6-bis-methoxycarbonyl-dihydropyrazin bzw. dessen Hydrierungsprodukte nachgewiesen.

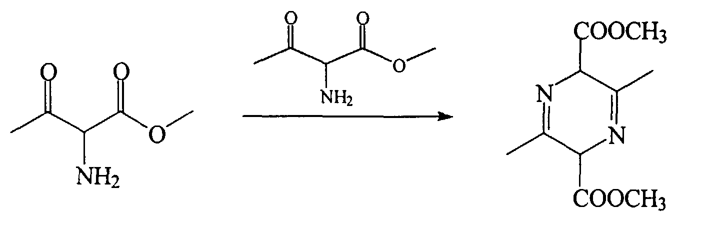

### Beispiel 2

Zu einer Mischung von 281 g Essigsäure und 984 g Acetessigsäuremethylester (8,48 mol) wurden bei 5 bis 10°C 375 g einer 40%igen wäßrigen Natriumnitritlösung (2,17 mol) in 3 Stunden unter Rühren und Kühlen zudosiert. Das Reaktionsgemisch wurde noch 5 Stunden unter Kühlung nachgerührt und dann auf Raumtemperatur erwärmen gelassen. Die klare Lösung wurde mit 4,7 g Palladium/Aktivkohle (5 % Pd) versetzt und bei 40°C unter 9 bis 10 bar Wasserstoffdruck so lange hydriert, bis kein Wasserstoff mehr aufgenommen wurde (etwa 24 Stunden). Die entstandene Suspension wurde zur Vervollständigung der Fällung mit 300 g Wasser versetzt und filtriert. Es wurden 399 g katalysatorhaltiger Filterrückstand erhalten (1,89 mol 2,4-Dimethyl-3,5-bis-methoxycarbonyl-pyrrol; Ausbeute: 87 % bezogen auf Natriumnitrit; 45 % bezogen auf Acetessigsäuremethylester).

## Patentansprüche

1. Verfahren zum Herstellen von 2,4-Dimethyl-3,5-bis-alkoxycarbonyl-pyrrol, bei dem ein Acetessigsäurealkylester zum 2-Nitroso-acetessigsäurealkylester nitrosiert, die Nitrosoverbindung zum Amin reduziert und das Amin zusammen mit unsubstituiertem Acetessigsäurealkylester unter Ringschluß zum 2,4-Dimethyl-3,5-bis-alkoxycarbonyl-pyrrol kondensiert wird, dadurch gekennzeichnet, daß man die Nitrosoverbindung mit Wasserstoff in Gegenwart eines Edelmetallkatalysators hydriert und die entstandene Aminoverbindung ohne Isolierung in Gegenwart des entsprechenden nicht nitrosierten Acetessigsäurealkylesters zum 2,4-Dimethyl-3,5-bis-alkoxycarbonyl-pyrrol kondensiert.

2. Verfahren nach Anspruch 1, dadurch gekennzeichnet daß man den Acetessigsäurealkylester mit 10 bis 50 % der stöchiometrischen Menge an salpetriger Säure nitrosiert und das erhaltene teilweise nitrosierte Gemisch unmittelbar der Kondensation unterwirft.

3. Verfahren nach Anspruch 1, dadurch gekennzeichnet, daß man einen Acetessigsäurealkylester mit 1 bis 4 C-Atomen in der Alkylgruppe einsetzt.

4. Verfahren nach Anspruch 1, dadurch gekennzeichnet, daß man als Edelmetall ein Metall der Platingruppe einsetzt.

5. Verfahren nach Anspruch 1, dadurch gekennzeichnet, daß man die katalytische Hydrierung bei einer zwischen Raumtemperatur und 60°C liegenden Temperatur durchführt.

6. Verfahren nach Anspruch 1, dadurch gekennzeichnet, daß man die katalytische Hydrierung unter einem Druck von 5 bis 20 bar durchführt.
